Europäisches Patentamt

European Patent Office

Office européen des brevets

(19)

(11) **EP 0 703 786 B1**

(12) **EUROPÄISCHE PATENTSCHRIFT**

(45) Veröffentlichungstag und Bekanntmachung des Hinweises auf die Patenterteilung:
**13.02.2002  Patentblatt 2002/07**

(51) Int Cl.⁷: **A61K 38/04**, A61K 38/09, A61P 31/18

(21) Anmeldenummer: **94913518.0**

(22) Anmeldetag: **02.04.1994**

(86) Internationale Anmeldenummer:
**PCT/EP94/01037**

(87) Internationale Veröffentlichungsnummer:
**WO 95/00168 (05.01.1995 Gazette 1995/02)**

(54) **VERWENDUNG VON NONA- UND DEKAPEPTIDEN ZUR HERSTELLUNG EINES ARZNEIMITTELS ZUR BEKÄMPFUNG VON AIDS**

USE OF NONA- AND DECAPEPTIDES IN THE PREPARATION OF A DRUG FOR THE TREATMENT OF AIDS

UTILISATION DE NONA- ET DECAPEPTIDES POUR LA FABRICATION D'UN MEDICAMENT CONTRE LE SIDA

(84) Benannte Vertragsstaaten:
**AT BE CH DE DK ES FR GB GR IE IT LI LU MC NL PT SE**

(30) Priorität: **18.06.1993  DE 4320201**

(43) Veröffentlichungstag der Anmeldung:
**03.04.1996  Patentblatt 1996/14**

(73) Patentinhaber: **Zentaris AG**
**60314 Frankfurt/Main (DE)**

(72) Erfinder:
• **ENGEL, Jürgen**
  **D-63755 Alzenau (DE)**

• **KUTSCHER, Bernhard**
  **D-63477 Maintal (DE)**
• **BERND, Michael**
  **D-60316 Frankfurt am Main (DE)**
• **NIEMEYER, Ulf**
  **D-63067 Offenbach (DE)**

(56) Entgegenhaltungen:
**EP-A- 0 192 492        EP-A- 0 299 402**
**EP-A- 0 335 726        WO-A-90/03980**
**WO-A-92/09626**

• **DIALOG,File 157:AIDSLINE, No. 93333809 Int.Conf. AIDS (GERMANY),June 6-11,1993 9(1), p195 (abstr.no. PO-A18-0362)**

**Beschreibung**

Ab 1981 wurde bei homosexuellen Männern in New York und in San Franzisco gehäuft bislang seltene unheilbare Infektionen beobachtet. Bei allen Patienten fand man schwere Defekte der Immunabwehr. Bald danach wurde von Luc Montagnier am Institut Pasteur ein neues Virus, das humane Immunschwäche-Virus (human immunodeficiency virus, HIV) entdeckt.

Zur Therapie von Aids ist zur Zeit nur das Didesoxynucleosid-Analoge 3'-Azido-3'-desoxythymidin (AZT) oder Zidovudine (INN) zugelassen. Es vermag die Krankheitssymptome zu lindern und die mittlere Überlebenszeit der AIDS-Patienten zu verlängern (EP-A 206 497). Unter AZT-Theraple kommt es zur Ausbildung resistenter Virusstämme und zu gravierenden Nebenwirkungen auf das Knochenmark. Weitere Medikamente befinden sich in der klinischen Erprobung. So beschreibt DE-OS 39 35 580 die Verwendung von 1-Octadecyl-2-methyl-glycero-3-phosphocholin zur Herstellung von Arzneimitteln zur Bekämpfung von HIV-Infektionen. EP-A 493 378 beschreibt die Verwendung von 2'-3'-Dedeoxyguanosin oder von Mono- oder Triphosphaten von 2'-3'-Dedeoxyguanosin für den gleichen Zweck. Alle diese Sustanzen befinden sich noch in der klinischen Entwicklung und haben die Marktreife noch nicht erreicht.

Die auf dem Gebiet der antiviralen Chemotherapie eingesetzten Arzneimitteln weisen gegenwärtig nicht die wünschenswerte Selektivität auf. Es besteht also ein hoher Bedarf an gut verträglichen, hochwirksamen Medikamenten, die nicht nur den Verlauf der Krankheit verzögern, sondern die auch die Vermehrung der Viren unterbinden und darüber hinaus in der Lage sind, das geschwächte Immunsystem der Patienten zu stabilisieren

Es wurde nun überraschend gefunden, daß im Aids-Screening-Versuch an CEM-IW-Zellen die LHRH-analogen Dekapeptide gemäß den Formeln II - VIII eine anti-HIV sowie eine wachstumsstimulierende Wirkung auf Zellkulturen haben.

Gegenstand der Erfindung ist die Herstellung eines Arzneimittels auf der Basis von peptidischen LHRH-Antagonisten,

Die Verbindungen sind selbst bei der höchsten eingesetzten Dosierung wenig toxisch.

Die Aminosäuresequenz des LHRH lautet:

$$\text{LHRH} = \text{p-Glu}^1\text{-His}^2\text{-Trp}^3\text{-Ser}^4\text{-Tyr}^5\text{-Gly}^6\text{-Leu}^7\text{-Arg}^8\text{-}$$

$$\text{Pro}^9\text{-Gly}^{10}\text{-NH}_2$$

Die allgemeine Formel I beschreibt die erfindungsgemäßen Peptide:

<u>Formel I</u>: Ac-D-Nal(2)-D-Phe(4-Cl)-xxx-A-

-B-yyy-zzz-Arg-C-D-Ala-NH$_2$

wobei gilt:

xxx =　　D-Pal(3), D-Phe(4-Cl), D-Trp, mit der Maßgabe, daß wenn xxx D-Trp ist, yyy D-Cit bedeutet

yyy =　　D-Cit, D-Lys(R), D-Arg, D-Hci

　　　　wobei R ($C_1$-$C_4$)-Acyl und ($C_1$-$C_{10}$)-Alkyl ist

zzz =　　L-Leu, Nle, Nva, t-Leu

A =　　Ser, Ser(Zucker)

　　　　wobei der Zuckerrest Glucose, Galactose, Allose, Altrose, Mannose, Gulose, Idose und Talose ist

B =　　Tyr, Lys(Nic), Mop

C =　　Pro, Ala

sowie die pharmazeutisch akzeptablen Salze der Peptide wie beispielsweise Hydrochlorid, Trifluoracetat, Acetat, Sulfat, Phosphat, Mesylat oder Tosylat.

Die verwendbaren Abkürzungen für Aminosäuren, Peptide und ihre Derivate sind von der IUPAC-IUB-Commission für Biochemische Nomenklatur empfohlen.
(European J. Biochem, 1984, <u>138</u>, 9-37)

Abkürzungen für weniger gebräuchliche Aminosäuren:

| | |
|---|---|
| Dpa bedeutet | 2,3-Diaminopropionsäure |
| Nal bedeutet | 3-(2-Naphthyl)-alanin, |
| Thi bedeutet | β-2'-Thienylalanin, |
| Tpi bedeutet | 2,3,4,9-Tetrahydro-1H-pyrido-[3,4-b]-indol-3-carbonsäure |
| Nic bedeutet | Nicotinoyl |
| Mop bedeutet | 4-(Morpolinomethyl)-phenylalanin |

Besonders bevorzugte Verbindungen gemäß der allgemeinen Formel I stellen folgende Aminosäuresequenzen dar:

$$\text{Formel II} = [\text{Ac-DNal(2)}^1, \text{D-Phe(pCl)}^2, \text{D-Pal(3)}^3,$$

$$\text{D-Cit}^6, \text{Nle}^7, \text{D-Ala}^{10}]\text{-LHRH}$$

$$\text{Formel III} = [\text{Ac-DNal(2)}^1, \text{D-Phe(pCl)}^2, \text{D-Pal(3)}^3,$$

$$\text{D-Cit}^6, \text{Nva}^7, \text{D-Ala}^{10}]\text{-LHRH}$$

$$\text{Formel IV} = [\text{Ac-DNal(2)}^1, \text{D-Phe(pCl)}^2, \text{D-Trp}^3,$$

$$\text{D-Cit}^6, \text{D-Ala}^{10}]\text{-LHRH}$$

$$\text{Formel V} = [\text{Ac-DNal(2)}^1, \text{D-Phe(pCl)}^2, \text{D-Pal(3)}^3,$$

$$\text{D-Cit}^6, \text{D-Ala}^{10}]\text{-LHRH}$$

$$\text{Formel VI} = [\text{Ac-DNal(2)}^1, \text{D-Phe(pCl)}^2, \text{D-Pal(3)}^3,$$

$$\text{D-HCi}^6, \text{D-Ala}^{10}]\text{-LHRH}$$

$$\text{Formel VII} = [\text{Ac-DNal(2)}^1, \text{D-Phe(pCl)}^2, \text{D-Pal}^3,$$

$$\text{D-Cit}^6, \text{t-Leu}^7, \text{D-Ala}^{10}]\text{-LHRH}$$

$$\text{Formel VIII} = [\text{Ac-DNal(2)}^1, \text{D-Phe(pCl)}^2, \text{D-Pal(3)}^3,$$

$$\text{D-Cit}^6, \text{Ala}^9, \text{D-Ala}^{10}]\text{-LHRH}$$

Die erfindungsgemäßen Oligopeptide werden gemäß konventionellen literaturbekannten Verfahren synthetisiert. Eine zusammenfassende Beschreibung der dabei in Frage kommenden Verfahren findet sich beispielsweise in M. Bodanszky, Principles of Peptide Synthesis, Springer-Verlag, Berlin, Heidelberg, New York 1984.
Die Prinzipien der Festphasensynthese von Polypeptiden werden beispielsweise im Lehrbuch von J.M. Stewart und J.D. Young, Solid Phase Peptide Synthesis Pierce Chem. Co., Rockford, I1, 1984 (2. Auflage) und im Übersichtsartikel von L. Barany et al. , Int.J. Peptide Protein Res. 30, 705-739 (1987) erläutert.

Die Synthesen der Peptide gemäß den Formeln II - VIII gelangen gemäß Blockdiagramm an Methyl-Benzhydrylamin-Harz (Hydrochlorid-Handelsform) der Firma Advanced Chem. Tech/Louisville (Kentucky), USA, welches jeweils vor Anbindung des C-terminalen Boc-D-Alanins durch 10%iges Triethylamin in Dichlormethan(V/V) in die freie Base Überführt wurde.

Alle folgenden N$\alpha$-Boc-geschützten Aminosäuren wurden in dreifachem molaren Überschuß in Gegenwart von Diisopropylcarbodiimid (DIC) und 1-Hydroxybenzotriazol (HOBt) in $CH_2Cl_2$/DMF innerhalb von 90 Minuten gekuppelt, in D1-chlormethan/Dimethylformamid-Gemischen der Zusammensetzung 80/20 (V/V) und die Boc-Schutzgruppe durch halbstündige Einwirkung von 50 % Trifluoressigsäure (TFA) in Dichlormethan abgespalten. Reste freier Aminofunktionen wurden durch Acetylierung in fünffachem Überschuß an Acetylimidazol in Dichlormethan blockiert. Die Abfolge der Reaktionsschritte des Peptidaufbaus am Harz ergibt sich aus dem Blockdiagramm. Zur Abspaltung der harzgebundenen Peptide wurde das jeweilige Endprodukt der Festphasensynthese im Vakuum getrocknet und in 500facher Überschuß an HF/Anisol 10 : 1 (V/V) 45 bis 60 Minuten bei 0°C behandelt.

Nach Abdestillation von HF und Anisol im Vakuum fielen alle rohen Peptidamide durch Ausrühren mit wasserfreiem Ethylether als weiße Feststoffe an, die Abtrennung von mit anfallendem polymerem Träger erfolgte durch Auswaschen mit 50%iger (V/V)wäßriger Essigsäure. Durch schonendes Einengen der essigsauren Lösungen im Vakuum konnten die jeweiligen Peptide als hochviskose öle erhalten werden, welche sich nach Zugabe von absolutem Ether in der Kälte allmählich in weiße Feststoffe umwandelten. Die Rohpeptide wurden isoliert, auf einer Fritte mit absolutem Ether gewaschen und im Vakuum getrocknet.

Die präparative Aufreinigung erfolgte mit Hilfe von Hochdruckflüssigkeitschromatographie (HPLC) unter den angegebenen Bedingungen:

Parameter zur präparativen HPLC

| Geräte: | Pumpe Shimadzu LC-8A |
| | Detektor Shimadzu SPD-6A |
| | Integrator Shimadzu C-R4A |
| | Controller Shimadzu SCL-6A |

Reagenzien: Acetonitril Lichrosolv Merck Art. 30 Trifluoressigsäure Fluka Nr. 91700 Reinstwasser (Seralpur-Anlage)

| mobile Phase A: | 970 ml Wasser (VE-Wasser reinst) |
| | + 30 ml Acetonitril |
| | + 1 ml Trifluoressigsäure |

| mobile Phase B: | 300 ml Wasser (VE-Wasser, reinst) |
| | + 700 ml Acetonitril |
| | + 1 ml Trifluoressigsäure |

Flußrate: 40 ml/min

Druck: 14 bar

durchschnittliche Laufzeit eines präparativen
Durchlaufes: ca. 30 bis 40 min.

Die korrekte Identität aller synthetisierten Peptide wurde nach HPLC-Aufreinigung durch Aminosäureanalyse, Massenspektrometrie und $^1$H-NMR-Spektroskopie nachgewiesen.

Blockdiagramm zur Festphasensynthese der LH-RH-Peptide: Die sich wiederholenden Arbeitsgänge der Festphasensynthese sind im folgenden Schema zusammengefaßt; die Stufen 1 bis 17 beschreiben die notwendige Abfolge von Operationsschritten zur Ankupplung jeweils einer Aminosäure:

| Stufe | Reagentien und Operationen | Zelt [min] |
|---|---|---|
| 1 | Boc-Aminosäure, DIC, HOBt 1 : 1 : 1 | 90 |

(fortgesetzt)

| Stufe | Reagentien und Operationen | Zeit [min] |
|---|---|---|
| 2 | Farbtest auf vollständigen Umsatz, falls positiv: Wiederholung 1 und 2 | |
| 3 | MeOH, waschen | 1 |
| 4 | DIPEA, 10 % in $CH_2Cl_2$; Neutralisation | 2 |
| 5 | MeOH; waschen | 1 |
| 6 | $CH_2Cl_2$; waschen, 3-fach | 2 |
| 7 | $(CH_3CO)_2O$, Imidazol, 1 : 1, Acetylierung | 30 |
| 8 | MeOH; waschen, 2-fach | 1 |
| 9 | $CH_2Cl_2$; waschen, 3-fach | 2 |
| 10 | TFA, 50 % in $CH_2Cl_2$, Abspaltung Boc | 1 |
| 11 | TFA, 50 % in $CH_2Cl_2$, Abspaltung Boc | 30 |
| 12 | Isopropanol; waschen und entfärben | 2 |
| 13 | DIPEA, 10 % in $CH_2Cl_2$; Neutralisation | 3 |
| 14 | HeOH; waschen | 1 |
| 15 | DIPEA, 10 % in $CH_2Cl_2$; Neutralisation | 3 |
| 16 | MeOH; waschen | 1 |
| 17 | $CH_2Cl_2$; waschen, 2-fach | 2 |

Syntheseprodukt gemäß obigem Blockdiagramm:

Ac-D-Nal(2)-D-Phe(4Cl)-Xxx-A-B-Yyy-Zzz-Arg-Pro-D-Ala-$NH_2$ (gemäß Formel I)

Die Vollständigkeit der Synthese wurde mit dem Chloraniltest nach Th. Christensen; Acta Chem. Scand, B 33, 763 (1979) und mit dem Kaiser'schen Ninhydrintest nach Stewart, Young überprüft.

Die Erfindung betrifft ein Verfahren zur Herstellung eines Arzneimittels zur Therapie viraler Infektionen, vorzugsweise zur Behandlung von Aids. Weiter werden neue Peptide und ihre Synthese beschrieben, die zur antiviralen Therapie einsetzbar sind.

Die Peptide sind selbst bei der höchsten eingesetzten Dosierung wenig toxisch. Im Vergleich zur mit getesteten Referenzverbindung Azidothymidin liegen die $EC_{50}$-Werte im NCI-Versuch bei den untersuchten Peptiden zwischen 5,9 x $10^{-7}$ Mol/Liter und 2,0 x $10^{-5}$ Mol/Liter.

Die mitgeführte Referenzverbindung AZT (Azidothymidin) hatte beispielsweise einen EC50-Wert von 3,10 x $10^{-9}$ mol/l.

Alle Verbindungen wurden im Bereich von $10^{-4}$ bis $10^{-8}$ mol/l dosiert. Der üblicherweise angegebene IC50-Wert (inhibitorische Konzentration, bei der in der nicht infizierten Kultur 50 % Zellen absterben) ist daher größer als die höchste Dosierung.

Die behandelte infizierte Kultur weist einen EC-50-Wert von 4,5 x $10^{-5}$ mol/l auf. Da die Werte für die behandelte, nicht infizierte Kultur nicht auf einen IC50-Wert abfallen, kann für den IC50-Wert nur der Wert für die höchste Dosierung angegeben werden, das heißt IC50 = >3,3 x $10^{-5}$ mol/l. Der therapeutische Index (TI 50 = IC50/EC50) ist dann größer als 7,30.

Beschreibung der Screening-Methode auf Anti-HIV-Aktivität

Das Verfahren ist geeignet, Wirkstoffe aufzufinden, die in allen Phasen des Virusvermehrungszyklusses wirksam sind. Das Testprinzip beruht auf der Abtötung der T4-Lymphozyten durch das HIV-Virus.

Kleine Mengen an HIV-Virus werden den Zellkulturen zugesetzt. Es sind mindestens zwei vollständige virale Reproduktionszyklen erforderlich, um die T4-Lymphozyten abzutöten und um die Ergebnisse auswerten zu können.

Wirkstoffe, die mit Virionen (virusähnlichen Partikeln), Zellen oder mit Produkten der viralen Gene reagieren, um mit viralen Aktivitäten wechselzuwirken und so die Vermehrung der Viren zu blockieren, werden die Zellen vor dem Tod und vor der Lyse bewahren.

Um eine große Anzahl von mit Viren infizierter Zellen untersuchen zu können, 1st das Testsystem automatisiert. Verbindungen, die degenerieren, denaturieren oder die rasch metabolisiert werden, können jedoch mit der Testanordnung nicht sicher entdeckt werden.

Als Positivkontrolle für den Test dient AZT (Azidothymidin) und DDC.

Testverfahren

1. T4-Lymphozyten (CEM - Zelllinie) werden im Verhältnis Virus : Zelle von ca. 1 : 0,05 in Microtiterplatten mit Virus versetzt.

2. Die zu testende Substanz wird, wenn nicht anders angegeben, in Dimethylsulfoxid (DMSO) gelöst und im Verhältnis 1 : 200 (Gewichtsteile) verdünnt.

Weitere Verdünnungen werden in halblogarithmischen Schritten mit DMSO vorgenommen und dann sowohl auf infizierte wie auch auf nicht infizierte Zellkulturen gegeben.

3. Die Kulturen werden 6-7 Tage lang bei 37° C in einer 5%-$CO_2$-Atmosphäre inkubiert.

4. Das Tetrazolium-Salz XTT wird den Zellkulturen zugesetzt und die Zellkulturen werden weiter inkubiert, um die Formazan Farbreaktion durch Kuppeln mit Phenazinmethosulfonat (PMS) durch überlebende Zellen ablaufen zu lassen.

5. Die einzelnen Zellkulturen werden spektrophotometrisch analysiert und mikroskopisch auf überlebende Zellen untersucht, um den Schutzeffekt zu bestätigen.

6. Behandelte virusinfizierte Zellen werden mit behandelten, nicht infizierten Zellen verglichen. Weitere Vergleiche (unbehandelte, infizierte Zellen und unbehandelte, nicht infizierte Zellen, wirkstoffhaltige, zellfreie Vertiefungen) werden auf der gleichen Platte vorgenommen.

7. Die Aktivität der getesteten Verbindung wird bestimmt.

Die weiteren experimentellen Einzelheiten sind bei Weislow, J. Nat. Canc. Inst. 81 (8), Seite 577 (1989) zu entnehmen.

Damit eignen sich die erfindungsgemäßen Peptide zur Herstellung von Arzneimitteln zur Bekämpfung von Aids und zur Bekämpfung von Krankheiten, die mit der Infektion mit dem Immunschwächevirus verbunden sind (Aids related complex, ARC).

Dosierungshinweise:

Die Dosierung des erfindungsgemäßen Arzneimittels beträgt zwischen 0,01 mg und 10 mg bei täglicher Verabreichung.

Beispiel 1

Peptid gemäß der Formel II:

Massenspektrum: [M+H$^+$]= 1431

$^1$H-NMR-Spektrum (DMSO-d$_6$, 250 MHz) δ in ppm:

8,65-7,1; viele Multipletts, aromatische und NH-Signale; 7,0 und 6,6, 2,1 4H, aromatische H Tyr; 5,95 m, NHCONH$_2$ Cit; 4.8 -4,1, mehrere Multipletts, C$_d$H; 3,75 und 3,5, 2 Multipletts; aliphatische H;3,2-2,65, mehrere Multipletts, CβH aliphatische und aromatische Aminosäuren; 2,1-1,3, mehrere Multipletts, restliche aliphatische H; 1,780, d,3H, CH$_3$CO$^-$; 1,20, d,3H, CβHAla; 0,80, m, Nle

Beispiel 2

Peptid gemäß der Formel III:

$^1$H-NMR-Spektrum (DMSO-$d_6$, 250 MHz) δ in ppm:
8,65-7,1, viele Multipletts, aromatische und NH-Signale; 7,0 und 6,6 2d, 4H, aromatische H Tyr; 5,9 m, NHCONH$_2$, Cit; 4,8-4,1, mehrere Multipletts, C$_2$H; 3,75 und 3,5, 2 Multipletts, aliphatische H; 3,2-2,65, mehrere Multipletts, CβH aliphatische und aromatische Aminosäuren; 2,1-1,25, mehrere Multipletts, restliche aliphatische H; 1,70,5, 3H, CH$_3$CO$^-$; 1,20, d,3 H, CβH,Ala; 0,85,m 3H, Nva
Massenspektrum: [M+H$^+$]= 1417

Beispiel 3

Peptid gemäß der Formel IV:

Massenspektrum: [M+H$^+$]= 1469

$^1$H-NMR-Spektrum (DMSO-$d_6$, 250 MHz) δ in ppm:
8,2-6,5, viele Multipletts, aromatische und NH-Signale; 5,8 und 5,4, 2 m 3 H, NH-CO-NH$_2$ Citrollia, 4,5, 4,3, 4,2 und 4,0, Multipletts C$_d$H, 3,8- 2,6, mehrere Multipletts, aliphatische und aromatische CβH; 2.0-1,9, mehrere Multipletts, restlilche aliphatische Protonen; 1,6,5,3 H, CH$_3$CO$^-$; 1,1, d,2H, CβH Ala; 0,7, d,6 H CδH leu

Beispiel 4

Peptid gemäß der Formel V, INN: Cetrorelix

Massenspektrum: [M+H$^+$]= 1431

$^1$H-NMR-Spektrum (DMSO-$d_6$, 250 MHz) δ in ppm:
8,7-7,2, viele Multipletts, aromatische und NH-Signale; 7,05 und 6,65, 2d, 4H, aromatische H Tyr; 5,85, m NHCONH$_2$, Cit; 4,8-4,1, mehrere Multipletts, CαH; 3,8 und 3,55, 2 m, aliphatische H; 3,3-2,7 CβH aliphatische und aromatische Aminosäuren; 2,1-1,3, mehrere Multipletts, restliche aliphatische Signale; 1,7,5,3 H, CH$_3$CO$^-$; 1,2, d, 3 H, CβH Ala; 0,85 2d, 6H, CδH leu

Beispiel 5

Peptid gemäß der Formel VI:

Massenspektrum: [M+H$^+$]= 1444

$^1$H-NMR-Spektrum (DMSO-$d_6$, 250 MHz) δ in ppm: 8,6-7,1, viele Multipletts, aromatische und NH-Signale; 7,0 und 6,6, 2 d, 4 H, aromatische H Tyr; 4.8-4.0, mehrere Multipletts, CαH; 3,7 und 3,5, 2 m, 4 H; 3,2-2,7, mehrere Multipletts, CβH aromatische und aliphatische Aminosäuren; 2,0-1,0, mehrere Multipletts, restliche aliphatische Signale ; 1,7, 5, 3H, CH$_3$CO; 1,2,d, 3H, CβH Ala; 0,8, dd, 6H, CδH leu

Beispiel 6

Peptid gemäß der Formel VII:

Massenspektrum: [M+H$^+$]= 1431

$^1$H-NMR-Spektrum (DMSO-$d_6$, 250 MHz) δ in ppm:
8,6-7,1, viele Multipletts, aromatische und NH-Signale; 7,0 und 6,6, 2d, 4H, aromatische H Tyr; 5,9 m NHCONH$_2$, Cit; 4,8-4,0, mehrere Multipletts, CαH; 3,85 und 3,5, 2 Multipletts, aliphatische H; 3,1-2,7 mehrere Multipletts CβH aliphatische und aromatische Aminosäuren; 2,1-1,3, mehrere Multipletts, restliche aliphatische H; 1,7,5,3 H, CH$_3$CO$^-$; 1,20, d, 3H, CβH Ala; 0,85, 5, 9H, t-Butyl, Tle

Beispiel 7

Peptid gemäß der Formel VIII

Massenspektrum: [M+H$^+$]= 1405

$^1$H-NMR-Spektrum (DMSO-d$_6$, 500 MHz) $\delta$ in ppm:
9,1-7,3, viele Multipletts, aromat. und NH-Signale; 7,21, dd,4H, aromatische H p-cl-Phe; 7,0 und 6,6, 2d, 4 H, aromatische H Tyr; 5,9 und 5,4,2m, 3H, NHCONH$_2$ Cit; 4,7-4,1, mehrere Multipletts, c$\alpha$H; 3,55-2,8, mehrere Multipletts, C$\beta$H aliphatische und aromatische Aminosäuren; 1,70,5, 3H, CH$_3$CO$^-$; 1,55 und 1,45,2 m aliphatische Signale Arg und Leu; 0,80, dd, 6H, C$\delta$H Leu

## Patentansprüche

**1.** Verwendung von mindestens einem Peptid mit einer Aminosäuresequenz gemäß der allgemeinen Formel I

<u>Formel I</u>: Ac-D-Nal(2)-D-Phe(4-Cl)-xxx-A-

-B-yyy-zzz-Arg-C-D-Ala-NH$_2$

wobei gilt:

xxx =    D-Pal(3), D-Phe(4-Cl), D-Trp

yyy =    D-Cit, D-Lys(R), D-Arg, D-Hci

wobei R (C$_1$-C$_4$)-Acyl und (C$_1$-C$_{10}$)-Alkyl ist

zzz =    L-Leu, Nle, Nva, t-Leu

A =    Ser, Ser(Zucker)

wobei der Zuckerrest Glucose, Galactose, Allose, Altrose, Mannose, Gulose, Idose und Talose ist

B =       Tyr, Lys(Nic), Mop

C =       Pro, Ala, mit der Maßgabe, daß wenn C = Ala ist, das Peptid die Aminosäuresequenz

Ac-D-Nal(2)$^1$ - D-Phe(4-Cl)$^2$ - D-Pal(3)$^3$ -

Ser$^4$ - Tyr$^5$ - D-Cit$^6$ - Leu$^7$- Arg$^8$ - Ala$^9$- D-Ala$^{10}$

besitzt
oder einem pharmazeutisch annehmbaren Salz davon, wie beispielsweise einem Hydrochlorid, Trifluoracetat, Acetat, Sulfat, Phosphat, Mesylat oder Tosylat, zur Herstellung eines Arzneimittels zur Bekämpfung von AIDS und/ oder ARC.

**2.** Verwendung des Peptids mit der Aminosäuresequenz gemäß Formel II

Ac-D-Nal(2)$^1$ - D-Phe(4-Cl)$^2$ - D-Pal(3)$^3$ -

Ser$^4$ - Tyr$^5$ - D-Cit$^6$ - Nle$^7$ - Arg$^8$ - Pro$^9$- D-Ala$^{10}$

oder einem pharmazeutisch annehmbaren Salz davon zur Herstellung eines Arzneimittels zur Bekämpfung von AIDS und/oder ARC.

**3.** Verwendung des Peptids mit der Aminosäuresequenz gemäß Formel III

$$\text{Ac-D-Nal(2)}^1 \text{ - D-Phe(4-Cl)}^2 \text{ - D-Pal(3)}^3 \text{ -}$$

$$\text{Ser}^4 \text{ - Tyr}^5 \text{ - D-Cit}^6 \text{ - Nva}^7 \text{ - Arg}^8 \text{ - Pro}^9 \text{- D-Ala}^{10}$$

oder einem pharmazeutisch annehmbaren Salz davon zur Herstellung eines Arzneimittels zur Bekämpfung von AIDS und/oder ARC.

**4.** Verwendung des Peptids mit der Aminosäuresequenz gemäß Formel IV

$$\text{Ac-D-Nal(2)}^1 \text{ - D-Phe(4-Cl)}^2 \text{ - D-Trp}^3 \text{ -}$$

$$\text{Ser}^4 \text{ - Tyr}^5 \text{ - D-Cit}^6 \text{ - Leu}^7 \text{ - Arg}^8 \text{ - Pro}^9 \text{- D-Ala}^{10}$$

oder einem pharmazeutisch annehmbaren Salz davon zur Herstellung eines Arzneimittels zur Bekämpfung von AIDS und/oder ARC.

**5.** Verwendung des Peptids mit der Aminosäuresequenz gemäß Formel V

$$\text{Ac-D-Nal(2)}^1 \text{ - D-Phe(4-Cl)}^2 \text{ - D-Pal(3)}^3 \text{ -}$$

$$\text{Ser}^4 \text{ - Tyr}^5 \text{ - D-Cit}^6 \text{ - Leu}^7 \text{ - Arg}^8 \text{ - Pro}^9 \text{- D-Ala}^{10}$$

oder einem pharmazeutisch annehmbaren Salz davon zur Herstellung eines Arzneimittels zur Bekämpfung von AIDS und/oder ARC.

**6.** Verwendung des Peptids mit der Aminosäuresequenz gemäß Formel VI

$$\text{Ac-D-Nal(2)}^1 \text{ - D-Phe(4-Cl)}^2 \text{ - D-Pal(3)}^3 \text{ -}$$

$$\text{Ser}^4 \text{ - Tyr}^5 \text{ - D-Hci}^6 \text{ - Leu}^7 \text{ - Arg}^8 \text{ - Pro}^9 \text{- D-Ala}^{10}$$

oder einem pharmazeutisch annehmbaren Salz davon zur Herstellung eines Arzneimittels zur Bekämpfung von AIDS und/oder ARC.

**7.** Verwendung des Peptids mit der Aminosäuresequenz gemäß Formel VII

$$\text{Ac-D-Nal(2)}^1 \text{ - D-Phe(4-Cl)}^2 \text{ - D-Pal(3)}^3\text{-}$$

$$\text{Ser}^4 \text{ - Tyr}^5 \text{ - D-Cit}^6 \text{ - t-Leu}^7\text{- Arg}^8 \text{ - Pro}^9 \text{- D-Ala}^{10}$$

oder einem pharmazeutisch annehmbaren Salz davon zur Herstellung eines Arzneimittels zur Bekämpfung von AIDS und/oder ARC.

**8.** Verwendung des Peptids mit der Aminosäuresequenz gemäß Formel VIII

$$\text{Ac-D-Nal(2)}^1 \text{ - D-Phe(4-Cl)}^2 \text{ - D-Pal(3)}^3\text{-}$$

$$\text{Ser}^4 \text{ - Tyr}^5 \text{ - D-Cit}^6 \text{ - Leu}^7 \text{ - Arg}^8 \text{ - Ala}^9 \text{- D-Ala}^{10}$$

oder einem pharmazeutisch annehmbaren Salz davon zur Herstellung eines Arzneimittels zur Bekämpfung von AIDS und/oder ARC.

**9.** Peptid mit einer Aminosäuresequenz gemäß der allgemeinen Formel I

<u>Formel I</u>: Ac-D-Nal(2)-D-Phe(4-Cl)-xxx-A-

-B-yyy-zzz-Arg-C-D-Ala-NH$_2$

wobei gilt:

xxx =   D-Pal(3), D-Phe(4-Cl), D-Trp

yyy =   D-Cit, D-Lys(R), D-Arg, D-Hci

wobei R $(C_1\text{-}C_4)$-Acyl oder $(C_1\text{-}C_{10})$-Alkyl ist

zzz =   L-Leu, Nle, Nva, t-Leu

A =   Ser, Ser(Zucker)

wobei der Zuckerrest Glucose, Galactose, Allose, Altrose, Mannose, Gulose, Idose und Talose ist

B =   Tyr, Lys(Nic), Mop

C =   Pro, Ala, mit der Maßgabe, daß wenn C = Ala ist, das Peptid die Aminosäuresequenz

$$\text{Ac-D-Nal(2)}^1 - \text{D-Phe(4-Cl)}^2\text{-D-Pal(3)}^3 -$$

$$\text{Ser}^4 - \text{Tyr}^5 - \text{D-Cit}^6 - \text{Leu}^7 - \text{Arg}^8 - \text{Ala}^9 - \text{D-Ala}^{10}$$

besitzt

oder ein pharmazeutisch annehmbares Salze davon, wie beispielsweise ein Hydrochlorid, Trifluoracetat, Acetat, Sulfat, Phosphat, Mesylat oder Tosylat, ausgenommen die folgenden Peptide

$$\text{Ac-D-Nal(2)}^1\text{-D-Phe(4-Cl)}^2\text{-(D-Pal(3) or D-Trp)}^3\text{-}$$

$$\text{Ser}^4\text{-Tyr}^5\text{-(D-Cit or D-Hci)}^6\text{-Leu}^7\text{-Arg}^8\text{-Pro}^9\text{-D-Ala}^{10}\text{-NH}_2.$$

**10.** Peptid mit der Aminosäuresequenz gemäß der allgemeinen Formel II

$$\text{Ac-D-Nal(2)}^1 - \text{D-Phe(4-Cl)}^2 - \text{D-Pal(3)}^3 -$$

$$\text{Ser}^4 - \text{Tyr}^5 - \text{D-Cit}^6 - \text{Nle}^7 - \text{Arg}^8 - \text{Pro}^9 - \text{D-Ala}^{10}$$

oder ein pharmazeutisch annehmbares Salz davon.

**11.** Peptid mit der Aminosäuresequenz gemäß der allgemeinen Formel III

$$\text{Ac-D-Nal(2)}^1 - \text{D-Phe(4-Cl)}^2 - \text{D-Pal(3)}^3 -$$

$$\text{Ser}^4 - \text{Tyr}^5 - \text{D-Cit}^6 - \text{Nva}^7 - \text{Arg}^8 - \text{Pro}^9 - \text{D-Ala}^{10}$$

oder ein pharmazeutisch annehmbares Salz davon.

12. Peptid mit der Aminosäuresequenz gemäß der allgemeinen Formel VII

$$\text{Ac-D-Nal(2)}^1 \text{ - D-Phe(4-Cl)}^2 \text{ - D-Pal(3)}^3 \text{ -}$$

$$\text{Ser}^4 \text{ - Tyr}^5 \text{ - D-Cit}^6 \text{ - t-Leu}^7 \text{ - Arg}^8 \text{ - Pro}^9 \text{- D-Ala}^{10}$$

oder ein pharmazeutisch annehmbares Salz davon.

13. Peptid mit der Aminosäuresequenz gemäß der allgemeinen Formel VIII

$$\text{Ac-D-Nal(2)}^1 \text{ - D-Phe(4-Cl)}^2 \text{ - D-Pal(3)}^3 \text{ -}$$

$$\text{Ser}^4 \text{ - Tyr}^5 \text{ - D-Cit}^6 \text{ - Leu}^7 \text{ - Arg}^8 \text{ - Ala}^9 \text{- D-Ala}^{10}$$

oder ein pharmazeutisch annehmbares Salz davon.

14. Arzneimittel, enthaltend mindestens ein Peptid gemäß einem der Ansprüche 9-13 oder ein pharmazeutisch annehmbares Salz davon, zur Bekämpfung von AIDS und/oder ARC.

**Claims**

1. Use of at least one peptide having an amino acid sequence of the general formula I

Formula I: Ac-D-Nal(2)-D-Phe(4-Cl)-xxx-A-

-B-yyy-zzz-Arg-C-D-Ala-NH$_2$

where:

xxx =     D-Pal(3), D-Phe(4-Cl), D-Trp

yyy =     D-Cit, D-Lys(R), D-Arg, D-Hci

where R is $(C_1$-$C_4)$-acyl and $(C_1$-$C_{10})$-alkyl

zzz =     L-Leu, Nle, Nva, t-Leu

A =     Ser, Ser(sugar)

where the sugar residue is glucose, galactose, allose, altrose, mannose, gulose, idose and talose

B =       Tyr, Lys(Nic), Mop

C =       Pro, Ala, with the proviso that, when C is Ala, the peptide has the amino acid sequence

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-Leu}^7\text{-Arg}^8\text{-Ala}^9\text{-D-Ala}^{10}$$

or a pharmaceutically acceptable salt thereof, such as, for example, a hydrochloride, trifluoroacetate, acetate, sulphate, phosphate, mesylate or tosylate, for producing a medicinal product for controlling Aids and/or ARC.

2. Use of the peptide having the amino acid sequence of formula II

$$Ac\text{-}D\text{-}Nal(2)^1\text{-}D\text{-}Phe(4\text{-}Cl)^2\text{-}D\text{-}Pal(3)^3\text{-}$$

$$Ser^4\text{-}Tyr^5\text{-}D\text{-}Cit^6\text{-}Nle^7\text{-}Arg^8\text{-}Pro^9\text{-}D\text{-}Ala^{10}$$

or a pharmaceutically acceptable salt thereof for producing a medicinal product for controlling Aids and/or ARC.

3. Use of the peptide having the amino acid sequence of formula III

$$Ac\text{-}D\text{-}Nal(2)^1\text{-}D\text{-}Phe(4\text{-}Cl)^2\text{-}D\text{-}Pal(3)^3\text{-}$$

$$Ser^4\text{-}Tyr^5\text{-}D\text{-}Cit^6\text{-}Nva^7\text{-}Arg^8\text{-}Pro^9\text{-}D\text{-}Ala^{10}$$

or a pharmaceutically acceptable salt thereof for producing a medicinal product for controlling Aids and/or ARC.

4. Use of the peptide having the amino acid sequence of formula IV

$$Ac\text{-}D\text{-}Nal(2)^1\text{-}D\text{-}Phe(4\text{-}Cl)^2\text{-}D\text{-}Trp^3\text{-}$$

$$Ser^4\text{-}Tyr^5\text{-}D\text{-}Cit^6\text{-}Leu^7\text{-}Arg^8\text{-}Pro^9\text{-}D\text{-}Ala^{10}$$

or a pharmaceutically acceptable salt thereof for producing a medicinal product for controlling Aids and/or ARC.

5. Use of the peptide having the amino acid sequence of formula V

$$Ac\text{-}D\text{-}Nal(2)^1\text{-}D\text{-}Phe(4\text{-}Cl)^2\text{-}D\text{-}Pal(3)^3\text{-}$$

$$Ser^4\text{-}Tyr^5\text{-}D\text{-}Cit^6\text{-}Leu^7\text{-}Arg^8\text{-}Pro^9\text{-}D\text{-}Ala^{10}$$

or a pharmaceutically acceptable salt thereof for producing a medicinal product for controlling Aids and/or ARC.

6. Use of the peptide having the amino acid sequence of formula VI

$$Ac\text{-}D\text{-}Nal(2)^1\text{-}D\text{-}Phe(4\text{-}Cl)^2\text{-}D\text{-}Pal(3)^3\text{-}$$

$$Ser^4\text{-}Tyr^5\text{-}D\text{-}Hci^6\text{-}Leu^7\text{-}Arg^8\text{-}Pro^9\text{-}D\text{-}Ala^{10}$$

or a pharmaceutically acceptable salt thereof for producing a medicinal product for controlling Aids and/or ARC.

7. Use of the peptide having the amino acid sequence of formula VII

$$Ac\text{-}D\text{-}Nal(2)^1\text{-}D\text{-}Phe(4\text{-}Cl)^2\text{-}D\text{-}Pal(3)^3\text{-}$$

$$Ser^4\text{-}Tyr^5\text{-}D\text{-}Cit^6\text{-}t\text{-}Leu^7\text{-}Arg^8\text{-}Pro^9\text{-}D\text{-}Ala^{10}$$

or a pharmaceutically acceptable salt thereof for producing a medicinal product for controlling Aids and/or ARC.

8. Use of the peptide having the amino acid sequence of formula VIII

$$Ac\text{-}D\text{-}Nal(2)^1\text{-}D\text{-}Phe(4\text{-}Cl)^2\text{-}D\text{-}Pal(3)^3\text{-}$$

$$\text{Ser}^4\text{-Tyr}^5\text{-D-Cit}^6\text{-Leu}^7\text{-Arg}^8\text{-Ala}^9\text{-D-Ala}^{10}$$

or a pharmaceutically acceptable salt thereof for producing a medicinal product for controlling Aids and/or ARC.

9. Peptide having an amino acid sequence of the general formula I

<u>Formula I</u>: Ac-D-Nal(2)-D-Phe(4-Cl)-xxx-A-

$$\text{-B-yyy-zzz-Arg-C-D-Ala-NH}_2$$

where:

xxx =     D-Pal(3), D-Phe(4-Cl), D-Trp

yyy =     D-Cit, D-Lys(R), D-Arg, D-Hci

          where R is $(C_1\text{-}C_4)$-acyl or $(C_1\text{-}C_{10})$-alkyl

zzz =     L-Leu, Nle, Nva, t-Leu

A =       Ser, Ser(sugar)

          where the sugar residue is glucose, galactose, allose, altrose, mannose, gulose, idose and talose

B =           Tyr, Lys(Nic), Mop

C =           Pro, Ala, with the proviso that, when C is Ala, the peptide has the amino acid sequence

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-Leu}^7\text{-Arg}^8\text{-Ala}^9\text{-D-Ala}^{10}$$

or a pharmaceutically acceptable salt thereof, such as, for example, a hydrochloride, trifluoroacetate, acetate, sulphate, phosphate, mesylate or tosylate, excepting the following peptides

$$\text{Ac-D-Nal(2)}^1\text{-D-Phe(4-Cl)}^2\text{-(D-Pal(3) or D-Trp)}^3\text{-}$$

$$\text{Ser}^4\text{-Tyr}^5\text{-(D-Cit or D-Hci)}^6\text{-Leu}^7\text{-Arg}^8\text{-Pro}^9\text{-}$$

$$\text{D-Ala}^{10}\text{-NH}_2.$$

10. Peptide having the amino acid sequence of the general formula II

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-Nle}^7\text{-Arg}^8\text{-Pro}^9\text{-D-Ala}^{10}$$

or a pharmaceutically acceptable salt thereof.

11. Peptide having the amino acid sequence of the general formula III

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-Nva}^7\text{-Arg}^8\text{-Pro}^9\text{-D-Ala}^{10}$$

or a pharmaceutically acceptable salt thereof.

**12.** Peptide having the amino acid sequence of the general formula VII

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-t-Leu}^7\text{-Arg}^8\text{-Pro}^9\text{-D-Ala}^{10}$$

or a pharmaceutically acceptable salt thereof.

**13.** Peptide having the amino acid sequence of the general formula VIII

$$\text{Ac-D-Nal(2)}^1\text{-D-Phe(4-Cl)}^2\text{-D-Pal(3)}^3\text{-}$$

$$\text{Ser}^4\text{-Tyr}^5\text{-D-Cit}^6\text{-Leu}^7\text{-Arg}^8\text{-Ala}^9\text{-D-Ala}^{10}$$

or a pharmaceutically acceptable salt thereof.

**14.** Medicinal product containing at least one peptide according to any of claims 9-13 or a pharmaceutically acceptable salt thereof for controlling Aids and/or ARC.

**Revendications**

**1.** Utilisation d'au moins un peptide ayant une séquence d'acides aminés selon la formule générale I

<u>Formule I :</u> Ac-D-Nal(2)-D-Phe-(4-Cl)xxx-A-B-yyy-zzz-Arg-C-D-Ala-NH$_2$

dans laquelle

xxx = D-Pal(3), D-Phe(4-Cl)), D-Trp
yyy = D-Cit, D-Lys(R), D-Arg, D-Hci,
R représente un acyle en $C_1$ à $C_4$ ou un alkyle en $C_1$ à $C_{10}$,
zzz = L-Leu, Nle, Nva, t-Leu,
A = Ser, Ser(sucre), le radical sucré étant le glucose, le galactose, l'allose, l'altrose, le mannose, le gulose, l'idose ou le talose,
B = Tyr, Lys(Nic), Mop
C = Pro, Ala, sous réserve que lorsque C = Ala, le peptide possède la séquence d'acides aminés Ac-D-Na (2)$^1$-D-Phe(4-Cl)$^2$-D-Pal(3)$^3$-Ser$^4$-Tyr$^5$-D-Cit$^3$-Leu$^7$-Arg$^8$-Ala$^9$-D-Ala$^{10}$

ou d'un de ses sels pharmaceutiquement acceptables des peptides comme par exemple le chlorhydrate, le trifluoroacétate, l'acétate, le sulfate, le phosphate, le mésylate ou le tosylate, pour la production d'un médicament visant à combattre le SIDA et/ou l'ARC.

**2.** Utilisation du peptide ayant la séquence d'acides aminés selon la formule II,

$$\text{Ac-D-Na(2)}^1\text{-D-Phe(4-Cl)}^2\text{-D-Pal(3)}^3\text{-Ser}^4\text{-Tyr}^5\text{-D-Cit}^3\text{-Leu}^7\text{-Arg}^8\text{-Pro}^9\text{-D-Ala}^{10}$$

ou d'un de ses sels pharmaceutiquement acceptables pour la production d'un médicament visant à combattre le SIDA et/ou l'ARC.

3. Utilisation du peptide ayant la séquence d'acides aminés selon la formule III,

$$\text{Ac-D-Na(2)}^1\text{-D-Phe(4-Cl)}^2\text{-D-Pal(3)}^3\text{-Ser}^4\text{-Tyr}^5\text{-D-Cit}^6\text{-Nva}^7\text{-Arg}^8\text{-Pro}^9\text{-D-Ala}^{10}$$

ou d'un de ses sels pharmaceutiquement acceptables de ce corps pour la production d'un médicament visant à combattre le SIDA et/ou l'ARC.

4. Utilisation du peptide ayant la séquence d'acides aminés selon la formule IV,

$$\text{Ac-D-Nal(2)}^1\text{-D-Phe(4-Cl)}^2\text{-D-Trp}^3\text{-Ser}^4\text{-Tyr}^5\text{-D-Cit}^6\text{-Leu}^7\text{-Arg}^8\text{-Pro}^9\text{-D-Ala}^{10}$$

ou d'un de ses sels pharmaceutiquement acceptables de ce corps pour la production d'un médicament visant à combattre le SIDA et/ou l'ARC.

5. Utilisation du peptide ayant la séquence d'acides aminés selon la formule V,

$$\text{Ac-D-Nal(2)}^1\text{-D-Phe(4-Cl)}^2\text{-D-Pal(3)}^3\text{-Ser}^4\text{-Tyr}^5\text{-D-Cit}^6\text{-Leu}^7\text{-Arg}^8\text{-Pro}^9\text{-D-Ala}^{10}$$

ou d'un de ses sels pharmaceutiquement acceptables de ce corps pour la production d'un médicament visant à combattre le SIDA et/ou l'ARC.

6. Utilisation du peptide ayant la séquence d'acides aminés selon la formule VI,

$$\text{Ac-D-Nal(2)}^1\text{-D-Phe(4-Cl)}^2\text{-D-Pal(3)}^3\text{-Ser}^4\text{-Tyr}^5\text{-D-Hci}^6\text{-Leu}^7\text{-Arg}^8\text{-Pro}^9\text{-D-Ala}^{10}$$

ou d'un de ses sels pharmaceutiquement acceptables de ce corps pour la production d'un médicament visant à combattre le SIDA et/ou l'ARC.

7. Utilisation du peptide ayant la séquence d'acides aminés selon la formule VII,

$$\text{Ac-D-Nal(2)}^1\text{-D-Phe(4-Cl)}^2\text{-D-Pal(3)}^3\text{-Ser}^4\text{-Tyr}^5\text{-D-Cit}^6\text{-t-Leu}^7\text{-Arg}^8\text{-Pro}^9\text{-D-Ala}^{10}$$

ou d'un de ses sels pharmaceutiquement acceptables de ce corps pour la production d'un médicament visant à combattre le SIDA et/ou l'ARC.

8. Utilisation du peptide ayant la séquence d'acides aminés selon la formule VIII,

$$\text{Ac-D-Nal(2)}^1\text{-D-Phe(4-Cl)}^2\text{-D-Pal(3)}^3\text{-Ser}^4\text{-Tyr}^5\text{-D-Cit}^6\text{-Leu}^7\text{-Arg}^8\text{-Ala}^9\text{-D-Ala}^{10}$$

ou d'un de ses sels pharmaceutiquement acceptables de ce corps pour la production d'un médicament visant à combattre le SIDA et/ou l'ARC.

9. Peptide ayant une séquence d'acides aminés selon la formule générale I

Formule I : Ac-D-Nal(2)-D-Phe-(4-Cl)-xxx-A-B-yyy-zzz-Arg-C-D-Ala-NH2

dans laquelle

xxx =  D-Pal(3), D-Phe(4-Cl)), D-Trp
yyy =  D-Cit, D-Lys(R), D-Arg, D-Hci,
où R  représente un acyle en $C_1$ à $C_4$ ou un alkyle en $C_1$ à $C_{10}$,

zzz =   L-Leu, Nle, Nva, t-Leu,
A =   Ser, Ser(sucre), le radical sucre étant le glucose, le galactose, l'allose, l'altrose, le mannose, le gulose, l'idose ou le talose,
B =   Tyr, Lys(Nic), Mop
C =   Pro, Ala, sous réserve que lorsque C = Ala, le peptide possède la séquence d'acides aminés

$$\text{Ac-D-Na(2)}^1\text{-D-Phe(4-Cl)}^2\text{-D-Pal(3)}^3\text{-Ser}^4\text{-Tyr}^5\text{-D-Cit}^3\text{-Leu}^7\text{-Arg}^8\text{-Ala}^9\text{-D-Ala}^{10}$$

ou d'un de ses sels pharmaceutiquement acceptables de ce corps, comme par exemple le chlorhydrate, le trifluoroacétate, l'acétate, le sulfate, le phosphate, le mésylate ou le tosylate, à l'exception des peptides suivants :

$$\text{Ac-D-Nal(2)}^1\text{-D-Phe(4-Cl)}^2\text{-(D-Pal(3) ou D-Trp)}^3\text{-Ser}^4\text{-Tyr}^5\text{-(D-cit ou D-Hci)}^6\text{-Leu}^7\text{-Arg}^8\text{-Pro}^9\text{-D-Ala}^{10}\text{-NH}_2$$

**10.** Peptide ayant la séquence d'acides aminés selon la formule générale II

$$\text{Ac-D-Na(2)}^1\text{-D-Phe(4-Cl)}^2\text{-D-Pal(3)}^3\text{-Ser}^4\text{-Tyr}^5\text{-D-Cit}^6\text{-Nle}^7\text{-Arg}^8\text{-Pro}^9\text{-D-Ala}^{10}$$

ou d'un de ses sels pharmaceutiquement acceptables.

**11.** Peptide ayant la séquence d'acides aminés selon la formule générale III,

$$\text{Ac-D-Na(2)}^1\text{-D-Phe(4-Cl)}^2\text{-D-Pal(3)}^3\text{-Ser}^4\text{-Tyr}^5\text{-D-Cit}^6\text{-Nva}^7\text{-Arg}^8\text{-Pro}^9\text{-D-Ala}^{10}$$

ou d'un de ses sels pharmaceutiquement acceptables.

**12.** Peptide ayant la séquence d'acides aminés selon la formule VII,

$$\text{Ac-D-Nal(2)}^1\text{-D-Phe(4-Cl)}^2\text{-D-Pal(3)}^3\text{-Ser}^4\text{-Tyr}^5\text{-D-Cit}^6\text{-t-Leu}^7\text{-Arg}^8\text{-Pro}^9\text{-D-Ala}^{10}$$

ou d'un de ses sels pharmaceutiquement acceptables.

**13.** Peptide ayant la séquence d'acides aminés selon la formule VIII,

$$\text{Ac-D-Nal(2)}^1\text{-D-Phe(4-Cl)}^2\text{-D-Pal(3)}^3\text{-Ser}^4\text{-Tyr}^5\text{-D-Cit}^6\text{-Leu}^7\text{-Arg}^8\text{-Ala}^9\text{-D-Ala}^{10}$$

ou d'un de ses sels pharmaceutiquement acceptables.

**14.** Médicament contenant au moins un peptide selon l'une des revendications 9 à 13 ou un de ses sels pharmaceutiquement acceptables, pour combattre le SIDA et/ou l'ARC.